(19) Europäisches Patentamt
European Patent Office
Office européen des brevets

(11) EP 0 501 713 B1

(12) EUROPEAN PATENT SPECIFICATION

(45) Date of publication and mention of the grant of the patent:
**02.05.1997 Bulletin 1997/18**

(51) Int Cl.$^{6}$: **C07D 261/08**, C07B 57/00, A61K 31/42

(21) Application number: **92301510.1**

(22) Date of filing: **24.02.1992**

# (54) Optically active aminoketone derivative and method for preparing same

Optisch aktive Aminoketone und Verfahren zu deren Herstellung

Aminocetones optiquement actifs et son procédé de préparation

(84) Designated Contracting States:
**AT BE CH DE DK ES FR GB IT LI NL SE**

(30) Priority: **26.02.1991 JP 30739/91**

(43) Date of publication of application:
**02.09.1992 Bulletin 1992/36**

(73) Proprietor: **MITSUI TOATSU CHEMICALS, Inc.**
**Chiyoda-Ku Tokyo 100 (JP)**

(72) Inventors:
- **Tanada, Hideki**
  **Mobara-shi, Chiba-ken (JP)**
- **Sakai, Kazuya**
  **Mobara-shi, Chiba-ken (JP)**
- **Kajiya, Seitaro**
  **Mobara-shi, Chiba-ken (JP)**
- **Ohto, Norio**
  **Mobara-shi, Chiba-ken (JP)**
- **Horikomi, Kazutoshi**
  **Mobara-shi, Chiba-ken (JP)**
- **Mizuchi, Akira**
  **Mobara-shi, Chiba-ken (JP)**

(74) Representative: **Stuart, Ian Alexander et al**
**MEWBURN ELLIS**
**York House**
**23 Kingsway**
**London WC2B 6HP (GB)**

(56) References cited:
**EP-A- 0 266 577** **EP-A- 0 414 391**

- **THE MERCK INDEX, 11th edition, 1989, page 262, edited by S. Budavari et al., Merck & Co., Inc., Rahway, US.**
- **JOURNAL OF THE AMERICAN CHEMICAL SOCIETY, 1966, pp.162-164**
- **AGRIC. BIOL. CHEM.,43 (2), 395-396, 1979.**

Remarks:
The file contains technical information submitted after the application was filed and not included in this specification

EP 0 501 713 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

The present invention relates to optically active 3-phenyl-5-[2-(1-pyrrolidinylmethyl)butyryl]isoxazole having a centrally acting muscle relaxant action and a method for preparing the same.

Heretofore, with regard to the optical resolution process of a compound having a centrally acting muscle relaxant action, techniques using an optical resolving agent are known. For example, Japanese Patent Application Laid-open No. 40779/1978 discloses a technique which comprises converting dl-tolperisone into two kinds of diastereomer salts by the use of optically active acetylphenylglycine as the resolving agent, separating these diastereomer salts mutually by the utilization of a difference of solubility thereof, and then recovering optically active tolperisone. In addition, Japanese Patent Application Laid-open Nos. 225367/1988 and 131171/1989 disclose a technique which comprises separating dl-2-methyl-1-(4-trifluoromethylphenyl)-3-pyrrolidino-1-propane by the use of optically active acetylphenylglycine or optically active malic acid as the resolving agent, and then isolating optically active 2-methyl-1-(4-trifluoromethylphenyl)-3-pyrrolidino-1-propane.

The present inventors have newly discovered that 3-phenyl-5-[2-(1-pyrrolidinylmethyl)butyryl]isoxazole has a centrally acting muscle relaxant action, and previously filed a patent application regarding this fact (Japanese Patent Application Laid-open No. 157375/1990). Afterward, further investigation has been continued, and as a result, it has been discovered that (+)-3-phenyl-5-[2-(1-pyrrolidinylmethyl)butyryl]isoxazole has a more remarkable central muscle relaxant action as compared with a racemic modification. However, a conventional technique cannot efficiently and optically resolve 3-phenyl-5-[2-(1-pyrrolidinylmethyl)butyryl]isoxazole.

An object of the present invention is to provide a (+) form of 3-phenyl-5-[2-(1-pyrrolidinylmethyl)butyryl]isoxazole by efficient optical resolution, and another object of the present invention is to provide an excellent central muscle relaxant.

In order to solve the above-mentioned problems, the present inventors have found that an optically active sulfonic acid, particularly optically active 10-camphorsulfonic acid is extremely effective as the resolving agent, and the present invention has been completed on the basis of this knowledge.

The present invention provides (+)-3-phenyl-5-[2-(1-pyrrolidinylmethyl)butyryl]isoxazole represented by the formula (1)

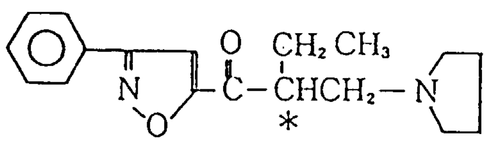

(1)

and a method for preparing optically active 3-phenyl-5-(2-(1-pyrrolidinylmethyl)butyryl]isoxazole and its salt which comprises the steps of reacting 3-phenyl-5-[2-(1-pyrrolidinylmethyl)butyryl]isoxazole with at least 2 equivalents of optically active 10-camphorsulfonic acid in a solvent; predominantly crystallizing one salt of the resultant two kinds of diastereomer salts out of the solvent to achieve resolution; and then demineralizing the respective diastereomer salts to isolate optically active 3-phenyl-5-[2-(1-pyrrolidinylmethyl)butyryl]isoxazole.

The preparation method of the present invention can be performed, for example, by the following procedure. That is, 3-phenyl-5-[2-(1-pyrrolidinylmethyl)butyryl]isoxazole is reacted with the optically active 10-camphorsulfonic acid in a suitable solvent to produce two kinds of diastereomer salts, and the solvent is then concentrated or cooled, or another solvent which can decrease solubility may be added, to crystallize the slightly soluble diastereomer salt and to thereby separate the solid. The resultant salts of optically active 3-phenyl-5-[2-(1-pyrrolidinylmethyl)butyryl]isoxazole and optically active 10-camphorsulfonic acid are demineralized by using a suitable alkali to isolate optically active 3-phenyl-5-[2-(1-pyrrolidinylmethyl)butyryl]isoxazole.

No particular restriction is put on the solvent which can be used in the present invention, so long as the solvent can dissolve 3-phenyl-5-[2-(1-pyrrolidinylmethyl)-butyryl]isoxazole and optically active 10-camphorsulfonic acid and which permits the formation of the diastereomer salts and which can dissolve the easily soluble and slightly soluble diastereomer salts between room temperature and their boiling points and which allows the slightly soluble diastereomer salt to crystallize by concentration, cooling or the addition of another solvent. Preferable examples of the solvent include ketones such as acetone, methyl ethyl ketone and methyl isobutyl ketone as well as alcohols such as methanol, ethanol, propanol and isopropanol, and particularly preferable examples of the solvent include alkyl acetates such as methyl acetate, ethyl acetate and butyl acetate. Above all, ethyl acetate is most preferable from the viewpoint of a resolution yield.

In this case, the amount of the solvent is in the wide range of from 1.0 to 50 times (V/W) as much as that of 3-phenyl-5-[2-(1-pyrrolidinylmethyl)butyryl]isoxazole, and preferably, it is in the range of from about 1 to about 30 times (V/W).

The ratio of 3-phenyl-5-[2-(1-pyrrolidinylmethyl)butyryl]isoxazole to optically active 10-camphorsulfonic acid, should be 2 or more equivalents of the latter per equivalent of the former, preferably 2 to 3 equivalents of the latter per equivalent of the former. In this case, when D-10-camphorsulfonic acid is used, (-)-3-phenyl-5-[2-(1-pyrrolidinylmethyl) butyryl]isoxazole crystallizes as a slightly soluble diastereomer salt, and when L-10-camphorsulfonic acid is used, (+)-3-phenyl-5-[2-(1-pyrrolidinylmethyl)butyryl]isoxazole crystallizes as a slightly soluble diastereomer salt.

No particular restriction is put on a temperature at which the diastereomer salt can be formed, but the temperature is preferably from room temperature to 50°C. A crystallization time is from about 1 to about 50 hours, preferably about 2 to about 30 hours.

Furthermore, the crystallization temperature should be below the boiling point or less of the solvent, and it is particularly preferably in the range of from -10 to 40°C.

Examples of the above-mentioned other solvent which can decrease the solubility include hexane and ethers.

The demineralization of the obtained diastereomer salt can be preferably carried out at a temperature of from 0 to 30°C in the presence of sodium bicarbonate, potassium bicarbonate, sodium carbonate, potassium carbonate, dilute sodium hydroxide, dilute potassium hydroxide, ammonia or the like in water. The amount of the alkali to be used should be 2 equivalents or more per equivalent of the diastereomer salt.

The isolation of optically active 3-phenyl-5-[2-(1-pyrrolidinylmethyl)butyryl]isoxazole from the resolving solution can be achieved by extracting the same with a non-hydrophilic solvent such as an ether, an alkyl acetate ester, chloroform, methylene chloride or hexane, and then distilling off the solvent.

The conversion of optically active 3-phenyl-5-[2-(1-pyrrolidinylmethyl)butyryl]isoxazole into an acid addition salt can be achieved by dissolving optically active 3-phenyl-5-[2-(1-pyrrolidinylmethyl)butyryl]isoxazole in an ether, an alkyl acetate ester, chloroform, methylene chloride, hexane or the like; adding 1 equivalent or more, preferably 1 to 10 equivalents of a mineral acid such as hydrochloric acid, nitric acid and sulfuric acid, or an organic acid such as acetic acid, oxalic acid, benzene-sulfonic acid or methanesulfonic acid; and then distilling off the solvent and the excess acid or collecting a crystallized salt of optically active 3-phenyl-5-[2-(1-pyrrolidinylmethyl)butyryl]isoxazole by filtration. Alternatively, the above-mentioned conversion into the acid addition salt can be achieved by extracting the above-mentioned organic solvent solution of optically active 3-phenyl-5-[2-(1-pyrrolidinylmethyl)butyryl]isoxazole with the above-mentioned mineral acid or organic acid; extracting the resultant aqueous solution again with an organic solvent such as chloroform in which an acid addition salt can be dissolved; distilling off the solvent from the solution or adding an organic solvent such as an ether, an alkyl acetate ester or hexane in which the acid addition salt of optically active 3-phenyl-5-[2-(1-pyrrolidinylmethyl)butyryl]isoxazole is scarcely soluble, to crystallize the acid addition salt; and then collecting the crystals by filtration.

The optically active compounds which can be obtained by the above-mentioned method are as follows:

(+)-3-phenyl-5-[2-(1-pyrrolidinylmethyl)butyryl]isoxazole and its salts.
(-)-3-phenyl-5-[2-(1-pyrrolidinylmethyl)butyryl]isoxazole and its salts.

The dose of the optically active aminoketone derivative of the present invention to each patient in need of a medical treatment is usually from 5 to 1000 mg, preferably from 10 to 300 mg to an adult per day, depending upon a symptom to be treated and an administration manner. The medicine can take any form of capsules, tablets, granules, syrup and powder which can be orally administered, and injections and suppositories which can be orally and parenterally administered. Examples of additives for the medicine include excipients (lactose, corn starch, sugar, sorbitol, calcium phosphate and the like), binders (syrup, gum arabic, gelatin, sorbitol, polyvinylpyrrolidone, hydroxypropyl cellulose and the like), glazing agents (magnesium stearate, talc, polyethylene glycol, silica and the like), disintegrators (potato starch, carboxymethyl cellulose and the like), and lubricants (sodium lauryl sulfate and the like). These additives can be used suitably in compliance with the form of the medicine.

Now, a compound of the present invention will be described in more detail in reference to reference examples and examples.

Reference Example 1

[3-phenyl-5-butyrylisoxazole]

10 g (0.082 mol) of benzaldoxime and 9 g (0.092 mol) of 1-hexine-3-ol were dissolved in 50 ml of dichloromethane. The resultant reaction solution was ice-cooled, and 58 g (0.1 mol) of a 12% aqueous sodium hypochlorite solution were added dropwise to the solution, while a solution temperature was maintained at 15 to 25°C. After the addition, the solution was stirred for 3 hours, while the solution temperature was maintained at 15 to 25°C.

49 g (0.079 mol) of the 12% aqueous sodium hypochlorite solution were added dropwise to the reaction solution. The temperature of the reaction solution was cooled to 10°C, and an aqueous pyridine hydrochloride solution (which

was prepared from 2.8 ml of 6 N hydrochloric acid and 1.3 ml of pyridine) was added dropwise thereto over 20 minutes. The reaction solution was stirred for 70 minutes, and 49 g (0.079 mol) of the 12% aqueous sodium hypochlorite solution were then added dropwise thereto.

The temperature of the solution was cooled to 10°C, and an aqueous pyridine hydrochloride solution (which was prepared from 1.4 ml of 6 N hydrochloric acid and 0.67 ml of pyridine) was added dropwise thereto over 10 minutes, followed by stirring for 70 minutes. 49 g (0.079 mol) of the 12% aqueous sodium hypochlorite solution were then added dropwise to this solution. The temperature of the solution was cooled to 10°C again, and the aqueous pyridine hydrochloride solution and the 12% aqueous sodium hypochlorite solution were similarly added.

100 ml of a 5% aqueous sodium hydrogensulfite solution were added to a dichloromethane layer obtained by separating the reaction solution, followed by stirring for 30 minutes. The dichloromethane layer obtained by separating the reaction solution was washed with 100 ml of water and 100 ml of a 1 N aqueous hydrochloric acid solution in this order. The washed dichloromethane layer was heated and concentrated to obtain a residue, and this residue was then recrystallized from 40 ml of ethanol, thereby obtaining 10.6 g (yield 59.6%) of the desired compound.
Melting point 89-90°C.

Reference Example 2

[3-phenyl-5-[2-(1-pyrrolidinylmethyl)butyryl]isoxazole hydrochloride]

20 g (93 mmol) of 3-phenyl-5-butyrylisoxazole and 7.93 g (111 mmol) of pyrrolidine were added to 62 g of methanol. To the solution, 9.04 g (111 mmol) of a 37% aqueous formalin solution were added dropwise, while the solution temperature was maintained at 20 to 30°C. After completion of the addition, the solution was stirred for 1 hour, while the solution temperature was maintained at 20 to 30°C.

178 g of ethyl acetate were added to the resultant reaction solution. 150 g of water were further added thereto, and separation and extraction were then carried out to obtain an organic layer. The obtained organic layer was ice-cooled, and 178 g of a 2 N aqueous hydrochloric acid solution were added, followed by separation and extraction. The resultant aqueous layer was extracted with 180 g of chloroform. The aqueous layer was extracted with 120 g of chloroform again, and the resultant chloroform layers were joined and dried over anhydrous sodium sulfate. This sodium sulfate was removed by filtration to obtain a chloroform solution, and 294 g of ethyl acetate were added dropwise to the chloroform solution with stirring. The solution was ice-cooled to precipitate crystals, and these crystals were then collected by filtration, washed with ethyl acetate, and then dried under reduced pressure to obtain 20.8 g (yield 67%) of the desired colorless compound.
Melting point 151-153°C

Reference Example 3

[3-phenyl-5-[2-(1-pyrrolidinylmethyl)butyryl]isoxazole]

125 g (0.37 mol) of 3-phenyl-5-[2-(1-pyrrolidinylmethyl)butyryl]isoxazole hydrochloride were added to a mixture of 400 ml of water and 300 ml of ethyl acetate. 620 ml of a 6% aqueous sodium hydrogencarbonate solution were further added dropwise to the solution under ice-cooling to precipitate crystals.

100 ml of ethyl acetate were added to this solution to dissolve the precipitated crystals, followed by separation, thereby obtaining an ethyl acetate layer. The aqueous layer was extracted with 500 ml of ethyl acetate again to obtain an ethyl acetate layer. These organic layers were joined, and then washed with 250 ml of water and 250 ml of a saturated sodium chloride solution. The ethyl acetate solution was dried with anhydrous sodium sulfate, and sodium sulfate was removed by filtration. The resultant filtrate was concentrated to dryness under reduced pressure, thereby obtaining 110 g (yield 98.9%) of the desired compound.
Melting point 68-69°C.

Example 1

[(+)-3-phenyl-5-[2-(1-pyrrolidinylmethyl)butyryl]isoxazole hydrochloride]

(1) (+)-3-phenyl-5-[2-(1-pyrrolidinylmethyl)butyryl]isoxazole L-10-camphorsulfonate

10 g (0.03 mol) of 3-phenyl-5-[2-(1-pyrrolidinylmethyl)butyryl]isoxazole hydrochloride were dissolved in a mixture of 60 ml of water and 70 ml of ethyl acetate. 72 ml of a 6% aqueous sodium hydrogencarbonate solution were added dropwise to the solution. The reaction solution was stirred for 10 minutes, followed by separation to obtain an organic

layer. The aqueous layer was extracted with 60 ml of ethyl acetate again, and the resultant organic layer was joined with the previously obtained organic layer, and the solution was then dried over anhydrous sodium sulfate. This sodium sulfate was then removed by filtration, and 14.2 g (0.06 mol) of L-10-camphorsulfonic acid {$[\alpha]^{20}_D$ = 21° (c = 2, water)} were added to the resultant filtrate, and they were stirred for 30 minutes to dissolve the same. The reaction solution was stirred for 6 hours under ice-cooling to precipitate crystals, and the crystals were collected by filtration, washed with ethyl acetate, and then dried under reduced pressure, thereby obtaining the desired (+)-3-phenyl-5-[2-(1-pyrrolidinylmethyl)butyryl]isoxazole L-camphor-sulfonate.
Yield (weight) 9.5 g
Yield (ratio) 42%
Melting point 115.8-116.3°C
$[\alpha]^{20}_D$ = -14.4° (c = 0.5, ethanol)
Optical purity 98.7% ee

(2) (+)-3-phenyl-5-[2-(1-pyrrolidinylmethyl)butyryl]isoxazole hydrochloride

9.4 g (12.3 mol) of (+)-3-phenyl-5-[2-(1-pyrrolidinylmethyl)butyryl]isoxazole L-10-camphorsulfonate were dissolved in a mixture of 54 ml of water and 54 ml of ethyl acetate. 30 ml of a 10% aqueous sodium carbonate solution were added dropwise to the solution. The reaction solution was stirred for 10 minutes, followed by separation to obtain an organic layer. The thus obtained organic layer was washed with 15 ml of a 10% aqueous sodium carbonate solution and 15 ml of water. 28 ml of a 2 N aqueous hydrochloric acid solution were added to this organic layer, followed by separation and extraction to obtain an aqueous layer. The organic layer was extracted with 15 ml of the 2 N aqueous hydrochloric acid solution again to obtain an aqueous layer, and this aqueous layer was joined with the previously obtained aqueous layer. 17.6 ml of chloroform were added to the joined aqueous solution, followed by extraction to obtain a chloroform layer. The aqueous layer was extracted with 17.6 ml of chloroform again, and the resultant chloroform layer was joined with the previously obtained chloroform layer. The thus obtained chloroform solution was then dried over anhydrous sodium sulfate. This sodium sulfate was then removed by filtration, 106 ml of ethyl acetate were added dropwise to the chloroform solution. This solution was stirred for 3 hours under ice-cooling. The precipitated crystals were collected by filtration, and then washed with ethyl acetate, thereby obtaining the desired (+)-3-phenyl-5-[2-(1-pyrrolidinylmethyl)butyryl]isoxazole hydrochloride.
Yield (weight) 3.4 g
Yield (ratio) 82%
Melting point 158-159.5°C
$[\alpha]^{20}_D$ = +29° (c = 0.5, water)
Optical purity 99.9% ee or more
NMR ($CDCl_3$, $\delta_{PPM}$); 0.99 (3H, t, J=7.3 Hz), 1.77-1.83 (1H, m), 1.88-1.97 (1H, m), 1.98-2.11 (2H, m), 2.11-2.23 (2H, m), 2.70-2.78 (1H, m), 2.79-2.95 (1H, m), 3.33 (1H, m), 3.47-3.64 (1H, m), 3.68-3.71 (1H, m), 3.83-3.87 (1H, m), 4.33-4.38 (1H, m), 7.48-7.50 (3H, m), 7.76 (1H, s), 7.87-7.90 (2H, m)

| Elemental Analysis ($C_{18}H_{22}N_2O_2$·HCl) | | | | |
|---|---|---|---|---|
| Calcd. | C:64.57, | H:6.92, | N:8.37, | Cl:10.59 |
| Found | C:64.51, | H:6.96, | N:8.20, | Cl:10.42 |

Example 2

[(-)-3-phenyl-5-[2-(1-pyrrolidinylmethyl)butyryl]isoxazole hydrochloride]

(1) (-)-3-phenyl-5-[2-(1-pyrrolidinylmethyl)butyryl]isoxazole butyryl]isoxazole D-10-camphorsulfonate

10 g (33.3 mmol) of 3-phenyl-5-[2-(1-pyrrolidinylmethyl)butyryl]isoxazole were dissolved in 200 ml of ethyl acetate. 11.5 g (67 mmol) of D-10-camphorsulfonic acid {$[\alpha]^{20}_D$ = +19.9° (c = 2, water)} were added to the resultant solution, followed by stirring for 30 minutes to dissolve the same. The solution was stirred for 2 hours under ice-cooling to precipitate crystals, and the crystals were collected by filtration, washed with ethyl acetate, and then dried under reduced pressure, thereby obtaining the desired (-)-3-phenyl-5-[2-(1-pyrrolidinylmethyl)butyryl]isoxazole D-camphorsulfonate.
Yield (weight) 11.8 g
Yield (ratio) 46%
Melting point 120-121°C

$[\alpha]^{20}_D$ = +17° (c = 0.5, ethanol)
Optical purity 96% ee

(2) (-)-3-phenyl-5-[2-(1-pyrrolidinylmethyl)butyryl]isoxazole hydrochloride

7.0 g (9.2 mmol) of (-)-3-phenyl-5-[2-(1-pyrrolidinylmethyl)butyryl]isoxazole D-10-camphorsulfonate were dissolved in a mixture of 40 ml of water and 40 ml of ethyl acetate. 22 ml of a 10% aqueous sodium carbonate solution were added thereto, followed by stirring for 10 minutes. The reaction solution was separated to obtain an organic layer. The thus obtained organic layer was washed with 11 ml of a 10% aqueous sodium carbonate solution. The organic layer was extracted with 17 ml of a 2 N aqueous hydrochloric acid solution twice and the resultant aqueous layers were then joined. This aqueous solution was extracted twice with 12 ml of chloroform and the chloroform layers were joined. The thus obtained chloroform solution was then dried over anhydrous sodium sulfate. This sodium sulfate was then removed by filtration, and 72 ml of ethyl acetate were added dropwise to the chloroform solution. The solution was then stirred at room temperature for 1 hour and further stirred for 1 hour under ice-cooling. The precipitated crystals were collected by filtration, thereby obtaining the desired (-)-3-phenyl-5-[2-(1-pyrrolidinylmethyl)butyryl]isoxazole hydrochloride.
Yield (weight) 2.5 g
Yield (ratio) 81%
Melting point 145-146°C
$[\alpha]^{20}_D$ = -27° (c = 0.5, water)
Optical purity 99.85% or more
NMR ($CDCl_3$, $\delta_{PPM}$); 0.99 (3H, t, J=7.3 Hz), 1.77-1.82 (1H, m), 1.88-1.98 (1H, m), 2.06-2.14 (2H, m), 2.14-2.24 (2H, m), 2.71-2.73 (1H, m), 2.81-2.88 (1H, m), 3.32 (1H, bd), 3.47 (1H, bs), 3.61-3.64 (1H, m), 3.66-3.69 (1H, m), 4.34-4.40 (1H, m), 7.48-7.52 (3H, m), 7.75 (1H, s), 7.89-7.91 (2H, m)

| Elemental Analysis ($C_{18}H_{22}N_2O_2$·HCl) | | | | |
|---|---|---|---|---|
| Calcd. | C:64.57, | H:6.92, | N:8.37, | Cl:10.59 |
| Found | C:64.65, | H:6.79, | N:8.03, | Cl:10.81 |

Example 3

[(+)-3-phenyl-5-[2-(1-pyrrolidinylmethyl)butyryl]isoxazole hydrochloride]

54 ml of water were added to a filtrate obtained when (-)-3-phenyl-5-[2-(1-pyrrolidinylmethyl)butyryl]isoxazole D-10-camphorsulfonate was collected by filtration in Example 2 (1), and 45 ml of a 10% aqueous sodium carbonate solution were added dropwise thereto. After,stirred for 10 minutes, the reaction solution was separated to obtain an organic layer. The obtained organic layer was washed with 20 ml of a 10% aqueous sodium carbonate solution, and further washed with 20 ml of water. 40 ml of a 2 N aqueous hydrochloric acid solution were added to the organic layer, followed by separation and extraction to obtain an aqueous layer. The organic layer was extracted with 30 ml of the 2 N aqueous hydrochloric acid solution again to obtain an aqueous layer, and this aqueous layer was then joined with the previously obtained aqueous layer. 40 ml of chloroform were added to the aqueous solution, followed by extraction to obtain a chloroform layer. 40 ml of chloroform were added to the aqueous layer and the solution was then extracted to obtain a chloroform layer, which was joined with the previously obtained chloroform layer. The thus obtained chloroform solution was dried over anhydrous sodium sulfate. This sodium sulfate was removed by filtration, and 240 ml of ethyl acetate were then added dropwise to the chloroform solution. This solution was stirred for 3 hours under ice-cooling. The precipitated crystals were collected by filtration and then washed with ethyl acetate to obtain 4.5 g (optical purity 87% ee) of the desired (+)-3-phenyl-5-[2-(1-pyrrolidinylmethyl)butyryl]isoxazole hydrochloride. 4.5 g of this hydrochloride were dissolved in 40 ml of chloroform. 120 ml of ethyl acetate were added dropwise to this solution, followed by stirring for 3 hours under ice-cooling. The precipitated crystals were collected by filtration, thereby obtaining the desired (+)-3-phenyl-5-[2-(1-pyrrolidinylmethyl)butyryl]isoxazole hydrochloride.
Yield (weight) 3.6 g
Yield (ratio) 59%
Melting point 158-159°C
$[\alpha]^{20}_D$ = +28° (c = 0.5, water)
Optical purity 99.5% ee

Evaluation Example

A centrally acting muscle relaxant action, an action to skeletal muscle afferent discharge and acute toxicity of an optically active aminoketone derivative of the present invention were confirmed by the following animal experiments.

1. Decerebrate rigidity remission action

Using the method suggested by Ono et al. [H. Ono et al., Gen. Pharmacol., 18, 57 (1987)], the rigidity remission action of the optically active aminoketone derivative of the present invention for the decerebrate rigidity induced by radio frequency lesions of rat brains was investigated.

(Procedure)

Each Wistar male rat (body weight: 300-400 g) was anesthetized with ether and then fixed on a stereotaxic apparatus. In accordance with the Pellegrino's stereotaxic brain atlas, the electrodes of a lesion generator (manufactured by Radionics Company) were punctuated to AP: 0, L: ±1.5, V: -3. While the electrodes were maintained at a tip temperature of 80°C, a high-frequency current of about 25 mA was applied for 180 seconds so that both left and right sites corresponding to brainstem cutting between the colliculus superior and the colliculus inferior were damaged. The rigidified rat was fixed in the abdominal position, and the tension of each extension reflex of the extensor of the hind limbs was recorded. Assuming that the tension before the administration was 100%, the rate of rigidity inhibition is expressed in terms of percentage:

$$(100 - \frac{\text{Tension after administration}}{\text{Tension before administration}} \times 100)$$

The test compounds were intravenously administered at 3 and 6 mg/kg. The results are set forth in Table 1.

Table 1

| | Test Compound | Dose (mg/kg, i.v.) | Rigidity Inhibition (%) |
|---|---|---|---|
| 1. | (+) form | 3 | 59 |
| | | 6 | 78 |
| 2. | (-) form | 3 | 33 |
| | | 6 | 53 |
| 3. | racemic form | 3 | 56 |
| | | 6 | 65 |

2. Action to skeletal muscle afferent discharge

The musculus triceps surae was separated from each of Wistar rats (9 to 10 weeks old; Japanese rats) under a mixed anesthetic of urethane and α-chlorarose. There were cut out all the branches of the sciatic nerve except the branch which controls the musculus triceps surae. The medulla spinalis was subjected to laminectomy, and roots of L6 or less were cut out. Next, L4 and L5 posterior roots on the same side were cut and separated from the central end, and L4 and L5 anterior roots were cut out for the purpose of eliminating the influence of the superior central. A nerve fiber coming from the musculus triceps surae was only separated from the L5 posterior root. A tension of 20 g was applied to the peripheral end of the musculus triceps surae, and each discharge frequency of the nerve was recorded. Assuming that the discharge frequency before the administration is 100%, the discharge frequency of the nerve was expressed in terms of percentage:

$$(100 - \frac{\text{Discharge frequency at most inhibition}}{\text{Discharge frequency before administration}} \times 100)$$

The test compounds were intravenously administered at 6 and 12 mg/kg. The results are set forth in Table 2.

Table 2

| | Test Compound | Dose (mg/kg, i.v.) | Discharge Frequency (%) |
|---|---|---|---|
| 1. | (+) form | 6<br>12 | 28<br>14 |
| 2. | (-) form | 6 | 83 |
| 3. | racemic form | 6<br>12 | 46<br>27 |

2. Acute Toxicity

ddy Male mice (body weights: 25-30 g) were used. Each test compound was intraperitoneally administered, and 72 hours later, the mice were observed as to whether they were alive or dead.

50% lethal doses $LD_{50}$ (mg/kg) were calculated. The results are set forth in Table 3.

Table 3

| | Test Compound | $LD_{50}$ (mg/kg) |
|---|---|---|
| 1. | (+) form | 386 |
| 2. | (-) form | 760 |
| 3. | racemic form | 445 |

According to the centrally acting muscle relaxant action (Table 1) represented by the decerebrate rigidity remission action, it is apparent that the (+) form is much more excellent as compared with the racemic form and the (-) form. Furthermore, the function for inhibiting the skeletal muscle afferent discharge is very effective to relax the abnormal tension of the muscle spindle. The (+) form is more excellent in the function for inhibiting the skeletal muscle afferent discharge (Table 2) as compared with the racemic form and the (-) form. With regard to the acute toxicity, the (+) form is substantially equal to the racemic form. In consequence, it is fair to say that (+)-3-phenyl-5-[2-(1-pyrrolidinylmethyl) butyryl]isoxazole is a very excellent centrally acting muscle relaxant.

## Claims

**Claims for the following Contracting States : AT, BE, CH, LI, DE, DK, FR, GB, IT, NL, SE**

**1.** (+)3-Phenyl-5-[2-(1-pyrrolidinylmethyl)butyryl] isoxazole represented by the formula (1) :

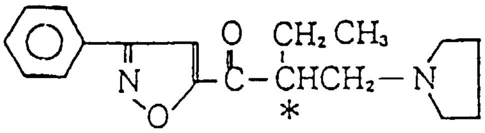

(1)

or a salt thereof.

**2.** An optical resolution method for resolving the racemic form of 3-phenyl-5-[2-(1-pyrrolidinylmethyl) butyryl]isoxazole, which comprises the steps of:

(a) reacting the racemic form with L-10-camphorsulfonic acid in a solvent in the molar ratio of 1 to at least 2 to obtain diastereomeric salts;

(b) crystallizing the salt of the (+) form of the isoxazole from the solvent;
(c) separating and collecting the crystals of the salt thus obtained from the solvent; and
(d) recovering the (+) form from the crystals by demineralization.

**3.** The optical resolution method according to claim 2 wherein the molar ratio is between 1:2 and 1:3.

**4.** The optical resolution methcd according to claim 2 or 3 wherein the solvent comprises an alkyl acetate ester.

**5.** An optical resolution method for resolving the racemic form of 3-phenyl-5-[2-(1-pyrrolidinylmethyl)butyryllisoxazole, which comprises the steps of:

(a) reacting the racemic form with D-10-camphorsulfonic acid in a solvent in the molar ratio of 1 to at least 2 to obtain diastereomeric salts;
(b) crystallizing the salt of the (-) form of the isoxazole from the mixture;
(c) separating the crystals from the mixture; and
(d) recovering the (+) form from the mixture.

**6.** The optical resolution method according to claim 5 wherein the molar ratio is between 1:2 and 1:3.

**7.** The optical resolution method according to claim 5 or 6 wherein the solvent comprises an alkyl acetate ester.

**8.** A centrally acting muscle relaxant containing (+)3-phenyl-5-[2-(1-pyrrolidinylmethyl)butyryl]isoxazole or its salt.

**9.** Use of a compound of claim 1 for the manufacture of a medicament for centrally acting muscle relaxation.

**10.** A pharmaceutical composition comprising (+)-3-phenyl-5-[2-(1-pyrrolidinylmethyl)-butyryl]isoxazole as obtainable by the method of any of claims 2-7, or a salt thereof.

**Claims for the following Contracting State : ES**

**1.** An optical resolution method for resolving the racemic form of 3-phenyl-5-[2-(1-pyrrolidinylmethyl) butyryl]isoxazole, which comprises the steps of:

(a) reacting the racemic form with L-10-camphorsulfonic acid in a solvent in the molar ratio of 1 to at least 2 to obtain diastereomeric salts;
(b) crystallizing the salt of the (+) form of the isoxazole from the solvent;
(c) separating and collecting the crystals of the salt thus obtained from the solvent; and
(d) recovering the (+) form from the crystals by demineralization.

**2.** The optical resolution method according to claim 1 wherein the molar ratio is between 1:2 and 1:3.

**3.** The optical resolution method according to claim 1 or 2 wherein the solvent comprises an alkyl acetate ester.

**4.** An optical resolution method for resolving the racemic form of 3-phenyl-5-[2-(1-pyrrolidinylmethyl)butyryl]isoxazole, which comprises the steps of:

(a) reacting the racemic form with D-10-camphorsulfonic acid in a solvent in the molar ratio of 1 to at least 2 to obtain diastereomeric salts;
(b) crystallizing the salt of the (-) form of the isoxazole from the mixture;
(c) separating the crystals from the mixture; and
(d) recovering the (+) form from the mixture.

**5.** The optical resolution method according to claim 4 wherein the molar ratio is between 1:2 and 1:3.

**6.** The optical resolution method according to claim 4 or 5 wherein the solvent comprises an alkyl acetate ester.

**7.** Use of (+)-3-phenyl-5-[2-pyrrolidinylmethyl)-butyryl]isoxazole or a salt thereof for the manufacture of a medicament

for centrally acting muscle relaxation.

## Patentansprüche

**Patentansprüche für folgende Vertragsstaaten : AT, BE, CH, LI, DE, DK, FR, GB, IT, NL, SE**

1. (+)-3-Phenyl-5-[2-(1-pyrrolidinylmethyl)butyryl]isoxazol, dargestellt durch die Formel (1):

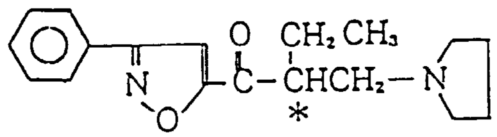

(1)

oder dessen Salz.

2. Optisch aktives Aufspaltungsverfahren zur Trennung der racemischen Form des 3-Phenyl-5-[2-(1-pyrrolidinylmethyl)-butyryl]isoxazol, daß die Schritte

(a) der Reaktion der racemischen Form mit L-10-Campferschwefelsäure in einem Lösungsmittel in dem molaren Verhältnis von 1 bis zu wenigstens 2, um diastereomere Salze zu erhalten,
(b) der Kristallisation des Salzes der (+)-Form des Isoxazols aus dem Lösungsmittel,
(c) der Trennung und der Vereinigung der Kristalle des so aus dem Lösungsmittel erhaltenen Salzes, und
(d) der Rückgewinnung der (+)-Form aus den Kristallen durch Demineralisierung,

umfaßt.

3. Optisch aktives Aufspaltungsverfahren nach Anspruch 2, bei dem das molare Verhältnis zwischen 1:2 und 1:3 liegt.

4. Optisch aktives Aufspaltungsverfahren nach Anspruch 2 oder 3, bei dem das Lösungsmittel Alkylacetatester umfaßt.

5. Optisch aktives Aufspaltungsverfahren zur Trennung der racemischen Form des 3-Phenyl-5-[2-(1-pyrrolidinylmethyl)butyryl]isoxazol, daß die Schritte

(a) der Reaktion der racemischen Form mit D-10-Campferschwefelsäure in einem Lösungsmittel in dem molaren Verhältnis von 1 bis zu wenigstens 2, um diastereomere Salze zu erhalten,
(b) der Kristallisation des Salzes der (-)-Form des Isoxazols aus der Mischung,
(c) der Trennung der Kristalle aus der Mischung, und
(d) der Rückgewinnung der (+)-Form aus der Mischung,

umfaßt.

6. Optisch aktives Aufspaltungsverfahren nach Anspruch 5, bei dem das molare Verhältnis zwischen 1:2 und 1:3 liegt.

7. Optisch aktives Aufspaltungsverfahren nach Anspruch 5 oder 6, bei dem das Lösungsmittel Alkylacetatester umfaßt.

8. Zentral wirkendes Muskelrelaxans enthaltend (+)-3-Phenyl-5-[2-(1-pyrrolidinylmethyl)butyryl]isoxazol oder seines Salzes.

9. Verwendung einer Verbindung nach Anspruch 1 für die Herstellung eines Medikaments zur zentral wirkenden Muskelentspannung.

10. Eine pharmazeutische Zusammensetzung umfassend (+)-3-Phenyl-5-[2-(1-pyrrolidinylmethyl)butyryl]isoxazol,

wie es durch das Verfahren nach einem der Ansprüche 2 bis 7 erhalten wird, oder dessen Salz.

**Patentansprüche für folgenden Vertragsstaat : ES**

1. Optisch aktives Aufspaltungsverfahren zur Trennung der racemischen Form des 3-Phenyl-5-[2-(1-pyrrolidinylmethyl)butyryl]isoxazol, daß die Schritte

   (a) der Reaktion der racemischen Form mit L-l0-Campferschwefelsäure in einem Lösungsmittel in dem molaren Verhältnis von 1 bis zu wenigstens 2, um diastereomere Salze zu erhalten,
   (b) der Kristallisation des Salzes der (+)-Form des Isoxazols aus dem Lösungsmittel,
   (c) der Trennung und der Vereinigung der Kristalle des so aus dem Lösungsmittel erhaltenen Salzes, und
   (d) der Rückgewinnung der (+)-Form aus den Kristallen durch Demineralisierung,

   umfaßt.

2. Optisch aktives Aufspaltungsverfahren nach Anspruch 1, bei dem das molare Verhältnis zwischen 1:2 und 1:3 liegt.

3. Optisch aktives Aufspaltungsverfahren nach Anspruch 1 oder 2, bei dem das Lösungsmittel Alkylacetatester umfaßt.

4. Optisch aktives Aufspaltungsverfahren zur Trennung der racemischen Form des 3-Phenyl-5-[2-(1-pyrrolidinylmethyl)butyryl]isoxazol, daß die Schritte

   (a) der Reaktion der racemischen Form mit D-10-Campferschwefelsäure in einem Lösungsmittel in dem molaren Verhältnis von 1 bis zu wenigstens 2, um diastereomere Salze zu erhalten,
   (b) der Kristallisation des Salzes der (-)-Form des Isoxazols aus der Mischung,
   (c) der Trennung der Kristalle aus der Mischung, und
   (d) der Rückgewinnung der (+)-Form aus der Mischung,

   umfaßt.

5. Optisch aktives Aufspaltungsverfahren nach Anspruch 4, bei dem das molare Verhältnis zwischen 1:2 und 1:3 liegt.

6. Optisch aktives Aufspaltungsverfahren nach Anspruch 4 oder 5, bei dem das Lösungsmittel Alkylacetatester umfaßt.

7. Verwendung von (+)-3-Phenyl-5-[2-(1-pyrrolidinylmethyl)butyryl]isoxazol oder dessen Salz für die Herstellung eines Medikaments zur zentral wirkenden Muskelentspannung.

**Revendications**

**Revendications pour les Etats contractants suivants : AT, BE, CH, LI, DE, DK, FR, GB, IT, NL, SE**

1. (+)3-phényl-5-[2-(1-pyrrolidinylméthyl)butyryl]isoxazole représenté par la formule (1):

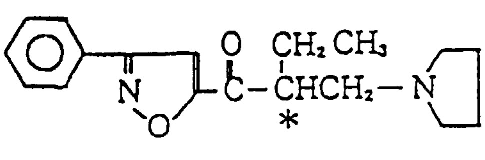

(1)

ou un de ses sels.

2. Procédé de dédoublement optique pour dédoubler la forme racémique du 3-phényl-5-[2-(1-pyrrolidinylméthyl)butyryl]isoxazole, qui comprend les étapes consistant:

(a) à faire réagir la forme racémique avec l'acide L-10-camphresulfonique dans un solvant dans le rapport molaire de 1 à au moins 2 pour obtenir des sels diastéréoisomères;
(b) à cristalliser le sel de forme (+) de l'isoxazole dans le solvant;
(c) à séparer et à recueillir les cristaux du sel ainsi obtenu d'avec le solvant; et
(d) à récupérer la forme (+) à partir des cristaux par déminéralisation.

**3.** Procédé de dédoublement optique selon la revendication 2, dans lequel le rapport molaire est compris entre 1:2 et 1:3.

**4.** Procédé de dédoublement optique selon la revendication 2 ou 3, dans lequel le solvant comprend un ester acétate d'alkyle.

**5.** Procédé de dédoublement optique pour dédoubler la forme racémique du 3-phényl-5-[2-(1-pyrrolidinylméthyl)butyryl]isoxazole, qui comprend les étapes consistant:

(a) à faire réagir la forme racémique avec l'acide D-10-camphresulfonique dans un solvant dans le rapport molaire de 1 à au moins 2 pour obtenir des sels diastéréoisomères;
(b) à cristalliser le sel de forme (-) de l'isoxazole dans le solvant;
(c) à séparer les cristaux d'avec le mélange; et
(d) à récupérer la forme (+) à partir du mélange.

**6.** Procédé de dédoublement optique selon la revendication 5, dans lequel le rapport molaire est compris entre 1:2 et 1:3.

**7.** Procédé de dédoublement optique selon la revendication 5 ou 6, dans lequel le solvant comprend un ester acétate d'alkyle.

**8.** Relaxant des muscles activés par le système central contenant le (+)3-phényl-5-[2-(1-pyrrolidinylméthyl)butyryl] isoxazole ou son sel.

**9.** Utilisation d'un composé selon la revendication 1 pour la fabrication d'un médicament destiné à la relaxation des muscles activés par le système central.

**10.** Composition pharmaceutique comprenant le (+)3-phényl-5-[2-(1-pyrrolidinylméthyl)butyryl]isoxazole qui peut être obtenu grâce au procédé selon l'une quelconque des revendications 2 à 7, ou un de ses sels.

**Revendications pour l'Etat contractant suivant : ES**

**1.** Procédé de dédoublement optique pour dédoubler la forme racémique du 3-phényl-5-[2-(1-pyrrolidinylméthyl)butyryl]isoxazole, qui comprend les étapes consistant:

(a) à faire réagir la forme racémique avec l'acide L-10-camphresulfonique dans un solvant dans le rapport molaire de 1 à au moins 2 pour obtenir des sels diastéréoisomères;
(b) à cristalliser le sel de forme (+) de l'isoxazole dans le solvant;
(c) à séparer et à recueillir les cristaux du sel ainsi obtenu d'avec solvant; et
(d) à récupérer la forme (+) à partir des cristaux par déminéralisation.

**2.** Procédé de dédoublement optique selon la revendication 1, dans lequel le rapport molaire est compris entre 1:2 et 1:3.

**3.** Procédé de dédoublement optique selon la revendication 1 ou 2, dans lequel le solvant comprend un ester acétate d'alkyle.

**4.** Procédé de dédoublement optique pour dédoubler la forme racémique du 3-phényl-5-[2-(1-pyrrolidinylméthyl)butyryl]isoxazole, qui comprend les étapes consistant:

(a) à faire réagir la forme racémique avec l'acide D-10-camphresulfonique dans un solvant dans le rapport molaire de 1 à au moins 2 pour obtenir des sels diastéréoisomères;
(b) à cristalliser le sel de forme (-) de l'isoxazole dans le solvant;
(c) à séparer les cristaux d'avec le mélange; et
(d) à récupérer la forme (+) à partir du mélange.

**5.** Procédé de dédoublement optique selon la revendication 4, dans lequel le rapport molaire est compris entre 1:2 et 1:3.

**6.** Procédé de dédoublement optique selon la revendication 4 ou 5, dans lequel le solvant comprend un ester acétate d'alkyle.

**7.** Utilisation du (+)-3-phényl-5-[2-(1-pyrrolidinylméthyl)butyryl]isoxazole ou un de ses sels pour la fabrication d'un médicament destiné à la relaxation des muscles activés par le système central.